# EUROPÄISCHE PATENTSCHRIFT

(11) **EP 0 758 745 B1**
(45) Veröffentlichungstag und Bekanntmachung des Hinweises auf die Patenterteilung: **11.08.1999**
(21) Anmeldenummer: 96108810.1
(22) Anmeldetag: 01.06.1996
(51) Int. Cl.: G01N 19/02, G01N 33/30

(54) **Verfahren zum Bestimmen des Beölungsgrades von Blechen und dgl. und Vorrichtung zur Durchführung des Verfahrens.**
Device and method for checking the oiliness of sheet metal and the like
Dispositif et méthode pour le contrôle de l'état graisseux des tôles ou des matériaux similaires

(30) Priorität: 14.08.1995 DE 19529879
(43) Veröffentlichungstag der Anmeldung: 19.02.1997
(73) Patentinhaber: Bayerische Motoren Werke Aktiengesellschaft, 80788 München (DE)
(72) Erfinder: Korus, Armin, 80935 München (DE); Heisler, Michael, 80798 München (DE)

(56) Entgegenhaltungen:
- EP-A- 0 476 747
- DE-A- 1 960 031
- FR-A- 2 427 606
- GB-A- 417 391
- PATENT ABSTRACTS OF JAPAN vol. 009, no. 207 (P-382), 24.August 1985 & JP-A-60 069533 (SUMITOMO KAGAKU KOGYO KK), 20.April 1985,

## Beschreibung

Die Erfindung bezieht sich auf ein Verfahren und eine Vorrichtung zum Bestimmen des Beölungsgrades von Blechen und dgl..

Diese Bestimmung ist beispielsweise im Rahmen der Fertigung von Kraftfahrzeugen von Bedeutung. Einerseits werden die Bleche während der Lieferung und/oder Lagerung vorübergehend beölt, um einen temporären Korrosionsschutz zu erreichen. Auch bei Umformungen werden die Bleche häufig mit Schmiermittel (Öle und Emulsionen) beschichtet. Andererseits werden die Bleche Klebeprozessen unterworfen. Damit eine einwandfreie Klebung erzielt werden kann, sind auf den Blechoberflächen abhängig vom Klebstoff und Blechwerkstoff nur geringe Ölmengen bis ca. 3 mg/m² zulässig.

Das einfachste Verfahren, eine einwandfreie Klebung zu erzielen, besteht darin, die verklebenden Teile vorher zu reinigen. Dies ist jedoch mit hohen Kosten verbunden. Auch die Möglichkeit, zerstörend Proben aus den Blechabschnitten zu entnehmen und mittels Gewichtsmessung auf die Ölmenge zurückzuschließen, ist aufwendig und wenig praktikabel. Es ist eine Vorrichtung der eingangs genannten Art bekannt, die auf einer spektroskopischen Analyse der Reflexion von Infrarotlicht beruht. Dazu wird eine Infrarotlichtquelle mit einer Frequenz überlagert und über ein Linsensystem auf die Blechoberfläche projiziert. Die reflektierten Lichtstrahlen werden von einer Empfängerdiode aufgenommen und weiterverarbeitet. Das spektroskopisch ermittelte Streuband der Reflexion erlaubt, durch Vergleich mit zuvor kalibrierten Daten die Ermittlung der auf der Oberfläche befindlichen Ölmenge (vgl. Bedienungsanleitung des Öldetektors SC3000 der Fa. Rautaruukki, New Technology, Version 4.01 vom 11.08.1993). Da das Reflexionsverhalten von metallischen Oberflächen sehr verschieden sein kann, muß das Meßgerät auf die Oberflächenstruktur und den Schmierstoff kalibriert werden. Das Meßgerät ist für den Einsatz in einer rauhen und schadstoffbelasteten Fabrikationshalle kaum geeignet, da unter den Störeinflüssen die Meßgenauigkeit deutlich leidet.

Aus der JP-A-60-069 533 ist ein Gerät bekannt, das zur Bestimmung der Reibung zwischen zwei offensichtlich aus dem gleichen Material bestehenden Folien dient. Die beiden aufeinanderliegenden Folien werden durch einen Meßstempel mit einem definierten Auflagegewicht belastet und durch einen Elektromotor gegeneinander solange verdreht, bis die zwischen den beiden bestehende Haftreibung überwunden wird. Das dabei auftretende maximale Drehmoment wird erfaßt. Aus dem Reibungskoeffizienten zweier Reaktionspartner, auch wenn sie nicht aus demselben, sondern aus unterschiedlichem Material bestehen, läßt sie jedoch keinesfalls auf den Beölungsgrad bzw. auf die auf einer Blech-Oberfläche liegende Ölmenge schließen.

Aus der FR-A-2 427 606 ist eine Vorrichtung zur Bestimmung der Qualität von Schmierölen bekannt, die bei der Verformung von Blechen zum Einsatz kommen.

Schließlich geht aus der GB-A- 417 391 hervor, die Schmiereigenschaften eines Öls zu überprüfen, indem es zwischen zwei Metallflächen eingebracht wird und die beiden Flächen dann gegeneinander gepreßt und verdreht werden.

Alle drei zuletzt genannten Dokumente zielen darauf ab, die qualitativen Eigenschaften eines Öls zu überprüfen. Aus qualitativen Aussagen lassen jedoch keine Rückschlüsse auf irgendwelche quantitativen Maße ableiten.

Der Erfindung liegt die Aufgabe zugrunde, ein Verfahren und eine zugehörige Vorrichtung zur quantitativen Bestimmung der auf einer Oberfläche liegenden Ölmenge zu schaffen, die sich durch einen einfachen und robusten Aufbau auszeichnet.

Die Erfindung löst diese Aufgabe für das Verfahren durch die Merkmale des Patentanspruchs 1.

Entsprechend dem Beölungsgrad unterliegt der Meßstempel bei seiner Drehbewegung einem unterschiedlichen Reibeinfluß. Bei hoher Beölung ist er leichtgängig, mit abnehmender Beölung wird er schwergängig. Dieses einfache Meßprinzip bietet die Gewähr dafür, daß die Vorrichtung selbst bei rauhen Einsatzbedingungen einwandfrei und betriebssicher arbeitet. Sie ist unempfindlich gegen Stoß, Erschütterung, Verschmutzung, Temperatur- und Feuchteschwankungen. Die Messung ist in allen Lagen, d.h. bei waagrechten oder senkrechten Flächen und auch bei engen Platzverhältnissen durchführbar.

Eine verfahrenstechnische Verbesserung der Erfindung ist Inhalt des Patentanspruchs 2, während vorteilhafte konstruktive Ausgestaltungen der Erfindung in den Patentansprüchen 3 und 4 angegeben sind.

Durch den elektrischen Antrieb wird die Wiederholgenauigkeit erhöht. Dieses Ziel wird noch besser erreicht, wenn die Drehbewegung mit einer definierten Geschwindigkeit erfolgt. Bringt man zusätzlich eine definierte Kraft auf, mit der der Stempel auf dem Blech aufliegt, so ist das Meßergebnis ein hervorragender Indikator für den Beölungsgrad.

Die Messung selbst kann auf unterschiedliche Weise vorgenommen werden. So kann beispielsweise der erforderliche Antriebsstrom des elektrischen Antriebs in seinem Verlauf aufgezeichnet werden und mit definierten Kennkurven für definierte unterschiedliche Beölungsgrade verglichen werden. Aus diesem Vergleich läßt sich dann der tatsächliche Beölungsgrad zweifelsfrei ermitteln.

Mit Hilfe des Antriebsstroms kann auch der Drehmomentverlauf während der Drehbewegung bestimmt werden. Auch hier bietet sich der Vergleich mit Kennkurven an. Das Maximum des Drehmoments bzw. des Antriebsstroms in seiner zeitlichen Lage und / oder in seiner Höhe liefert ein besonders gutes Maß für den Beölungsgrad. Bei diesem Maximum tritt der Übergang von Haften in Gleiten auf.

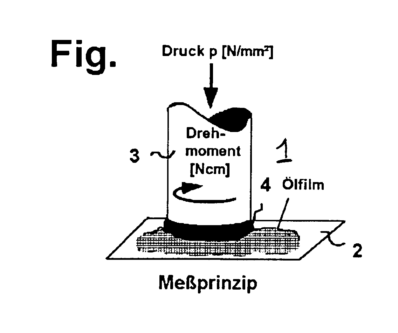

Anhand der Zeichnung ist die Erfindung weiter erläutert.

Die einzige Figur zeigt schematisch eine Vorrichtung 1 gemäß der Erfindung. In einem nicht dargestellten Gehäuse, das auf einem Blech 2 aufliegt, befindet sich ein kreisförmiger Meßstempel 3 drehbeweglich gelagert. Der Stempel 3 besitzt an seinem Ende eine Platte 4 aus definiertem Material, z.B. Gummi, die plan auf dem Blech 2 aufliegt. Mit dem Meßstempel 3 soll ein ggf. vorhandener Ölfilm 5, hier aus Gründen der Anschaulichkeit besonders deutlich dargestellt, untersucht und seine Dicke bestimmt werden. Aus der Dicke des Ölfilms läßt sich die auf dem Blech befindliche Ölmenge quantitativ bestimmen.

Für diese Bestimmung wird der physikalische Effekt genutzt, wonach sich bei steigender Ölmenge zwischen Grenzflächen die Reibzahl entsprechend reduziert. Die Reibzahl wird vorzugsweise über die Messung des Drehmoments bzw. des Reibmoments ermittelt. Der Meßstempel 3 wird mit einer definierten Kraft von beispielsweise 10 N belastet und mit konstanter Geschwindigkeit um die eigene Achse gedreht. Es wird die erforderliche Torsionskraft gemessen.

Bei zunehmender Ölmenge ergibt sich zwischen den Reibpartnem an der Grenzfläche Gummi und beölte Oberfläche ein reduziertes Reibmoment und umgekehrt. Dieser Wert korreliert mit der Ölmenge in dem praktisch relevanten Meßbereich von 0 bis rund 5 mg/m² Flächengewicht mit einer ausreichenden Genauigkeit.

Den Erfordemissen der Praxis kommt zugute, daß geringfügige Rauigkeitsschwankungen und/oder verschiedene Metalloberflächenwerkstoffe (Stahl, Stahlverzink, Alu) das Reibmoment nicht signifikant beeinflussen.

Der Antrieb des Meßstempels 3 kann je nach Erfordemis und Genauigkeitsanspruch mechanisch, elektromechanisch oder pneumatisch sein. Die Vorrichtung 1 selbst kann als Stand oder mobiles Handgerät ausgeführt sein. Für die Reproduzierbarkeit ist es notwendig, folgende Meßparameter festzulegen: Fläche, Form und Gummiqualität des Meßstempels 3, der Anpreßdruck (Quotient aus Anpreßkraft und Fläche des Meßstempels 3), Meßgeschwindigkeiten, -wege und -zeiten. Die Fläche des Meßstempels 3 kann klein dimensioniert sein. So hat sich ein Durchmesser von 8 bis 10 mm als ausreichend erwiesen. Damit können auch kleine Flächen (z. B. Flansche) gemessen werden.

## Patentansprüche

1. Verfahren zur quantitativen Bestimmung der auf einer Blech-Oberfläche befindlichen Ölmenge, bei der ein in einem Gehäuse drehbeweglich gelagerter und mit einer Platte aus Gummi-Material auf der Oberfläche aufliegender Meßstempel mittels eines elektrischen Antriebs einer Drehbewegung unterworfen wird, bei dem der Drehmomentverlauf während der Drehbewegung analysiert wird und bei dem ein Maß für die Ölmenge aus der Größe und/oder der zeitlichen Lage des Drehmoment-Maximums abgeleitet wird.

2. Verfahren nach Anspruch 1, dadurch gekennzeichnet, daß die Drehbewegung mit konstanter Geschwindigkeit vorgenommen wird.

3. Verfahren nach Anspruch 1 oder 2, dadurch gekennzeichnet, daß der Meßstempel während der Drehbewegung mit einer definierten Kraft belastet wird.

4. Vorrichtung zur Durchführung des Verfahrens nach einem der Ansprüche 1 bis 3, dadurch gekennzeichnet, daß der elektrische Antrieb ebenfalls im Gehäuse sitzt.

## Claims

1. A method for the quantitative determination of the quantity of oil present on a steel plate surface, in which a test plunger, mounted so as to be able to turn, in a casing and with a plate of rubber material lying on the surface, is subjected to a turning motion by means of an electrical drive, in which the torque during the turning motion is analysed and in which a measure of the oil quantity is derived from the magnitude and/or the duration of the maximum torque.

2. A method according to Claim 1, characterised in that the turning motion is undertaken at a constant speed.

3. A method according to Claim 1 or 2, characterised in that the test plunger is loaded with a defined force during the turning motion.

4. A device for the performance of the method according to any one of Claims 1 to 3, characterised in that the electric drive is also contained in the casing.

## Revendications

1. Procédé pour déterminer de façon quantitative la quantité d'huile qui se trouve sur la surface d'une tôle, selon lequel, le poinçon de mesure monté mobile en rotation dans un boîtier et appliqué avec une plaque en caoutchouc à la surface, est soumis à un mouvement de rotation par un moteur électrique, et selon lequel la courbe du couple pendant le mouvement de rotation est analysée pour en déduire une mesure de la quantité d'huile en fonction de la grandeur et/ou de la position dans le temps du maximum du couple.

2. Procédé selon la revendication 1,
caractérisé en ce que
le mouvement de rotation se fait à vitesse constante.

3. Procédé selon la revendication 1 ou 2,
caractérisé en ce que
le poinçon de mesure est chargé par une force déterminée pendant le mouvement de rotation.

4. Dispositif pour la mise en oeuvre du procédé selon l'une des revendications 1 à 3,
caractérisé en ce que
le moteur électrique se trouve également dans le boîtier.
